(19) Europäisches Patentamt
European Patent Office
Office européen des brevets

(11) Publication number: 0 442 612 A2

(12) **EUROPEAN PATENT APPLICATION**

(21) Application number: **91300368.7**

(22) Date of filing: **17.01.91**

(51) Int. Cl.5: **C07B 59/00**, C07F 9/09, C07D 317/22, C07C 43/178, C07C 43/23, C07C 43/166, C07C 43/215, C07F 9/6574

(30) Priority: **26.01.90 US 471259**

(43) Date of publication of application: **21.08.91 Bulletin 91/34**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Ponpipom, Mitree M.**
**431 Brookview Court**
**Branchburg, NJ 08876(US)**

(74) Representative: **Thompson, John Dr. et al**
**Merck & Co., Inc. European Patent Department Terlings Park Eastwick Road**
**Harlow, Essex CM20 2QR(GB)**

(54) **Process of making (3H)-labeled PAF preparations.**

(57) Disclosed is a process for making [$^3$H]-labelled-$C_{15-18}$PAF (Platelet Activating Factor). [$^3$H]-labelled-PAF is useful in numerous biomedical and biochemical assays such as the evaluation and comparison of known PAF antagonists and is exemplified by the following formula:

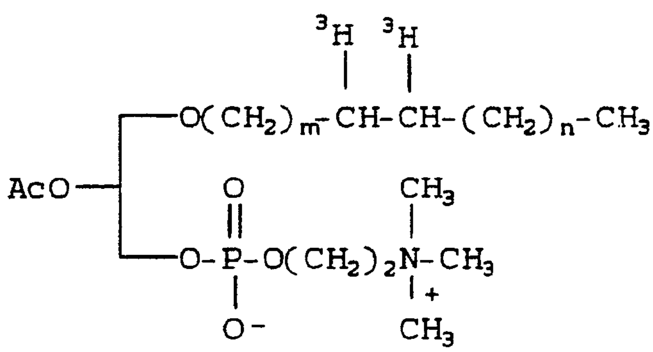

I

wherein m and n are positive integers and the sum of n + m is from 12 to 15.

EP 0 442 612 A2

# PROCESS OF MAKING $[^3H]$-LABELLED PAF PREPARATIONS

## BACKGROUND OF THE INVENTION

$[^3H]$-labelled PAF is widely recognized as an important tool in verifying, quantifying and otherwise evaluate known and exploratory PAF antagonists, an important class of antiinflammatory agents. Procedures for using $[^3H]$-labelled PAF are well known in the art and are exemplified by that provided in European Application 88202879.8 published June 28, 1989, herein incoporated by reference. Moreover, this reference discloses a number of useful PAG antagonists. These include 2-(5-methylsulfonyl)-4-n-propoxy-3-methoxyphenyl-5-(3,4,5-trimethoxyphenyltetrahydrofuran and trans-2-(5-(2-hydroxyethylsulfonyl)-4-n-propoxy-3-methoxyphenyl-5(3,4,5-trimethoxyphenyltetrahydrofuran. The $[^3H]$-labelled preparations are usually obtained by catalytic tritiation of the 1-alkenyl moieties of choline plasmalogens [C.A. Demopoulos et al, J. Biol. Chem., 254, 9355-9358 (1979)] or the alkenyl moieties of unsaturated PAF derived from shark liver oils [T. Muramatsu et al, Chem. Phys. Lipids. 29, 121-127 (1981)]. This process, which involves extensive chromatography, is very tedious and the purity of different batches may vary.

$[^3H]C_{16}$-PAF was previously prepared from racemic $\Delta^{9,10}$-hexadecenyl-PAF by the following transformations: treatment with phospholipase $A_2$, separation of the two lyso-$\Delta^{9,10}$-hexadecenyl-PAF isomers, re-acetylation and tritiation of the sn-isomer to give $[9',10'-{}^3H_2]C_{16}$-PAF [S.D. Wyrick et al., J. Lab. Compd. Radiopharm., 22, 1169-1174 (1985)]. The starting racemic $\Delta^{9,10}$-hexadecenyl-PAF was prepared in over 10 steps from glycerol [J. R. Surles et al., J. Med. Chem., 28, 73-78 (1985)].

Our invention differs from the prior art in that we use Chiral R-(-)-2,2-dimethyl-1,3-dioxolane-4-methanol (2,3-0-isopropylidene-L-glycerol) as a starting building block. The intermediates are also chiral and no enzyme is involved in the synthesis.

The description below utilized Fisher projections to indicate specific stereo chemistry.

## DETAILED DESCRIPTION OF THE INVENTION

The present invention encompasses a novel process for producing $[^3H]C_{15-18}$PAF.

The process encompassed by the invention may be represented as:

(A) Contacting a compound of formula J

$X(CH_2)R_1$ J

wherein:

$R_1$ is $C_{14-17}$alkenyl, and

X is a first leaving group such as O-methanesulfonate, O-P-toluenesulfonate, bromide or iodide;

in a first aprotic solvent such as N,N-dimethylformamide (DMF) or hexamethylphosphoramide (HMPA), with a strong base such as sodium hydride, potassium hydride, 1,5-diazabicyclo[3.4.0] nonene-5 (DBN), or 1,8-diazabicyclo[5.4.0] undecene-7 (DBU); and 2,3-O-isopropylidene- L-glycerol (IPG) to yield a compound of formula A:

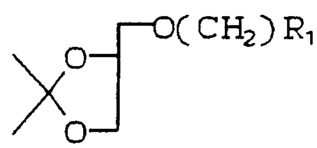

A

For purposes of the specification $C_{14-17}$ alkenyl is a linear alkenyl possessing one terminal or non-terminal double bond, such as $(CH_2)m\ -CH=CH(CH_2)_n - CH_3$, wherein m and n are positive integers and

the sum of m and n is 12 to 15. $C_{14-17}$ alkenyl can be, for example $\Delta^{9,10}$ or $\Delta^{11,12}$ $C_{14-17}$ alkenyl and more particularly $\Delta^{11,12}$ $C_{16}$ alkenyl.

Similarly, X, the first leaving group, is any moiety that can be readily displaced by the alkoxide ion of IPG. Such leaving groups include, but are not limited to sulfonylesters such as $C_{1-6}$ alkyl sulfonyloxy, and more particularly methanesulfonyloxy (MsO) or $C_{6-10}$ arylsulfonyloxy, and more particularly p-toluensulfonyloxy; and halides such as bromide or iodide.

Similarly, the first aprotic solvent, includes, but is not limited to DMF or HMPA.

Similarly, the strong base includes, but is not limited, to alkali metal or alkaline-earth metal hydroxides or hydride, as sodium or potassium hydroxide or hydride, organic heterocyclic nitrogen bases such as 1,5-diazabicyclo[3.4.0]nonene-5 (DBN), or 1,8-diazabicyclo[5.4.0]undecene-7 (DBU).

The molar ratio of $X(CH_2)R_1$ to IPG should be roughly 1:1 and, preferably from 0.7 - 1.5 to 1. The molar ratio of base to IPG should also be from 0.8 - 1.4 to 1, preferably 1.0-1.2 to 1.

Generally, the reaction is allowed to proceed from 0.5 to 8.0 hours, or overnight, until substantially complete. The reaction can be carried out at 0-100°C, preferably 0-30°C. Reactants are preferably added at 0-10°C.

The reaction can be carried out from 1-100 atmospheres of pressure and preferably at ambient pressure. The presence of oxygen is preferably minimized, such as by use of a nitrogen or other inert atmosphere.

After completion of the reaction step, product A may be purified by standard techniques such as extraction, evaporation and/or column chromotrography, prior to use in the next step.

(B) Contacting the compound of formula A, in alcohol, with a strong acid to yield a compound of formula B,

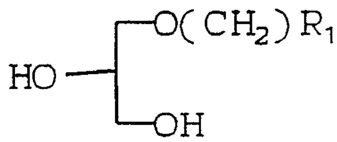

B

For purposes of the specification alcohols includes, but is not limited to $C_{1-8}$ alkanols, such as methanol and ethanol.

Similarly strong acids includes, but is not limited to, $C_{1-6}$alkylsulfonic acid such as methanesulfonic acid, $C_{6-10}$arylsulfonic acid such as p-tolunesulfonic acid, as well as mineral acids such as hydrochloric, sulfuric or phosphoric acid.

The molar ratio of acid to A is 0.05-0.25 to 1, preferably 0.15-0.25 to 1.

Generally, the reaction is allowed to proceed from 30 minutes to 3 days until substantially complete. The reaction can be carried out at 0-100°C, preferably 0-30°C.

The reaction can be carried out at up to 1-100 atmospheres of pressure and preferably at ambient pressure.

After completion of the reaction step, product B may be purified by standard techniques such as evaporation, extraction and/or column chromotography, prior to use in the next step.

(C) Contacting a compound of formula B, in a second aprotic solvent in the presence of a second base, with an acid labile protecting group to yield a compound of formula C,

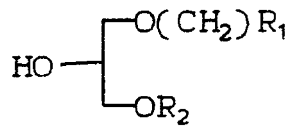

C

wherein $R_2$ is 4-methoxytrityl, trityl group or other acid labile protecting group.

For purposes of this specification the aprotic solvent includes but is not limited to moderately polar organic solvents such as halo$C_{1-6}$alkyl (e.g. dichloromethane), and ethers such as diethyl ether, di-n-butyl and diisopropyl ethers, anisole, cyclic ethers such as tetrahydropyran, 4-methyl-1,3-dioxane, dihydropyran, tetrahydrofurfuryl methyl ether, furan and 2-ethoxy-tetrahydrofuran. The second base is a moderate strength organic bases such as pyridine, triethylamine and the acid labile protecting group includes, but is not limited to, a 4-methoxytrityl or trityl group, from a reagent such as 4-methoxytrityl or trityl chloride or other halide.

The molar ratio of acid labile protecting group to B is approximately 0.5-2.0 to 1, preferrably 1.0-1.5 to 1.

Alternative protecting groups for use in Step (C) and the process steps described below are enumerated in Protective Groups in Organic Systhesis, Theodora W. Greene, Wiley and Sons pub. (1981).

Generally, the reaction is allowed to proceed from 0.5 to 8.0 hours until substantially complete. A reaction time of 3 hours, has been used advantageously, in order to ensure against loss of yield. The reaction can be carried out at 0-100° C, preferably 0-30° C.

The reaction can be carried out at up to 1-100 atmospheres of pressure, and preferably at ambient pressure. The presence of oxygen is preferably minimized, such as by use of a nitrogen or other inert atmosphere.

After completion of the reaction step, product C may be purified by standard techniques such as extraction, evaporation and/or column chromotrography, prior to use in the next step.

(D) Contacting a compound of formula C , in an organic solvent and in the presence of a third base, with a second protecting group such as methoxyethoxymethyl or methoxymethyl to yield a compound of formula D,

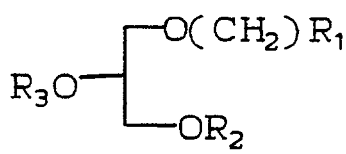

D

wherein $R_3$ is methoxyethoxymethyl or methoxymethyl or other protecting group, removable under near neutral conditions (e.g., $P^{H6\ 9}$).

For purposes of this specification the organic solvent includes but is not limited to tetrahydrofuran (THF) or dimethoxyethane or mono or poly halogenated $C_{1-6}$ alkane such as dichloromethane or chloroform. The third base includes, but is not limited to NaH or n-butyllithium or aliphatic amines such as mono, di, or tri $C_{1-6}$alkylamine including diisopropyl amine.

The second protecting group includes but is not limited to, such groups as methoxyethoxymethyl or methoxymethyl, from a reagent such as methoxyethoxymethyl or methoxymethyl chloride or other halide.

The molar ratio of third base to C is 1.0-4.0 to 1 preferably 2.0-3.0 to 1. The molar ratio of second protecting group to C is 1.0-4.0 to 1 preferably 2.0-3.0 to 1.

Generally, the reaction is allowed to proceed from 0.2 to 8.0 hours until substantially complete. The reaction can be carried out at 0-100 ° C, preferably 0-30 ° C.

The reaction can be carried out at up to 1-100 atmospheres of pressure, and preferably at ambient pressure. The presence of oxygen is preferably minimized, such as by use of a nitrogen or other inert atmosphere.

After completion of the reaction step, product D may be purified by standard techniques such as extraction, evaporation, and column chromotrography prior to use in the next step.

(E) Contacting a compound of formula D with a second acid, to yield a compound of formula E,

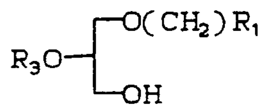

E

For purposes of this specification, the second acid includes, but is not limited to such organic acids as glacial acetic acid or formic acid containing varying amounts of water (e.g., 5 to 50%), as well as delute mineral acids such as hydrochloric, sulfuric and phosphoric acid. However the use of a mineral acid may increase the difficulty of controling the reaction.

Ordinarily the reaction is allowed to proceed from 0.5 to 8.0 hours until substantially complete. A reaction time of 3 hours, has been used advantageously, in order to ensure against loss of yield. The reaction can be carried out at 0-100 ° C, preferably 0-30 ° C.

The reaction can be carried out at up to 1-100 atmospheres of pressure, preferably at ambient pressure. The presence of oxygen is preferably minimized, such as by use of a nitrogen or other inert atmosphere.

(F) Converting a compound of formula E to yield a compound of formula F,

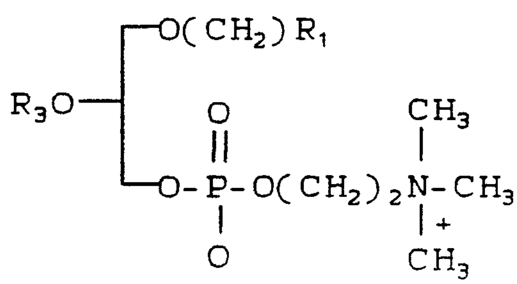

F

Step (F) may be satisfactorily performed by first contacting a compound of formula E, in a dry aromatic solvent, with triethylamine and 2-chloro-2-oxo-1,3-dioxa-2-phospholane to yield a compound of formula E',

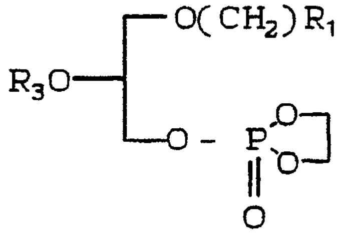

E'

In this step, any suitable aromatic solvent such as toluene, benzene or a xylene may be used in the reaction. Once again, the presence of oxygen is preferably minimized, such as by use of a nitrogen or other inert atmosphere. While the reaction may be allowed to proceed at room temperature, for convenience, reaction temperature is not considered critical. Similarly, while the reaction time is not considered critical, a reaction time of about 2 hours, has been used advantageously, in order to ensure against loss of yield.

Step (F) is then completed by contacting a compound of formula E', in a third aprotic polar solvent, with trimethylamine in acetonitrile to yield a compound of formula F.

In this second step, a third aprotic polar solvent such as acetonitrile or dioxane may be used in the reaction. While the reaction may be allowed to proceed overnight and at approximately 65 °C , for convenience, neither the temperature, pressure nor length of time is considered critical. The temperature, however, must be sufficient to ensure the completion of the reaction.

Alternative methods for carrying out step (F) are known to those skilled in the art. Two are outlined in the explanation accompanying Scheme I, infra.

(G) Contacting a compound of formula F, in a halogenated organic solvent, with $ZnBr_2$ or $TiCl_4$ to yield a compound of formula G,

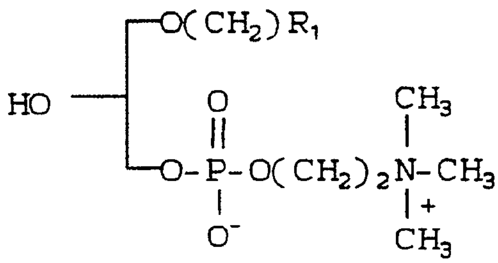

G

The ratio of $ZnBr_2$ or $TiCl_4$ to F is 0.05-1.0 to 1, preferably 0.1-0.5 to 1.

Generally the reaction is allowed to proceed from 0.5 to 8.0 hours, overnight, until substantially complete. The reaction can be carried out at 0-100 °C, preferably 0-30 °C.

The reaction can be carried out at up to 1-100 atmospheres of pressure, and preferably at ambient pressure.

(H) Contacting a compound of formula G, in a halogenated organic solvent and in the presence of a base with acetic anhydride to yield a compound of formula H,

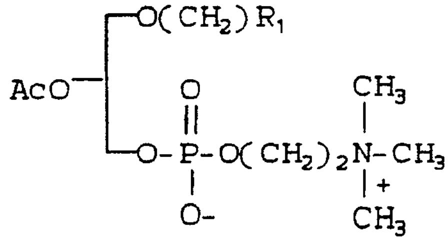

H

For purposes of the specification the fourth base, includes, but is not limited to 4-(N,N-dimethylamino)-pyridine or pyridine.

Generally the reaction is allowed to proceed from 0.5 to 8.0 hours, overnight, until substantially complete. The reaction can be carried out at 0-100° C, preferably 0-30° C.

The reaction can be carried out at up to 1-100 atmospheres of pressure, and preferably at under ambient pressure.

(I) Tritiating the compound of formula H at the point of unsaturation in the alkenyl group to yield a compound of formula I,

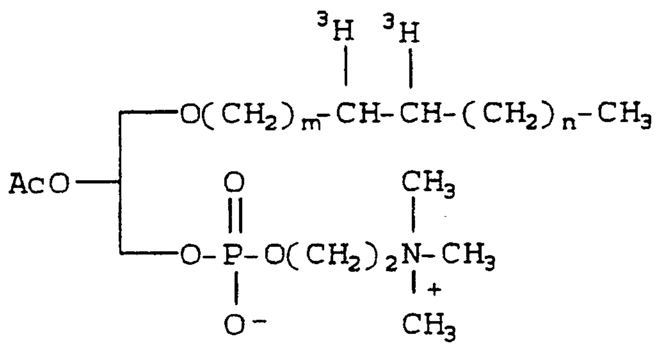

I

This step may be accomplished by well known procedures such as by catalytic tritiation of the 1-alkenyl moieties of choline plasmalogens [C.A. Demopoulos et al, J. Biol. Chem., 254, 9355-9358 (1979)].

A generalized description of the process is provided in Scheme 1. The reference numbers 1 through 11 provided in the Scheme, correspond to those provided in the Examples section.

## SCHEME 1

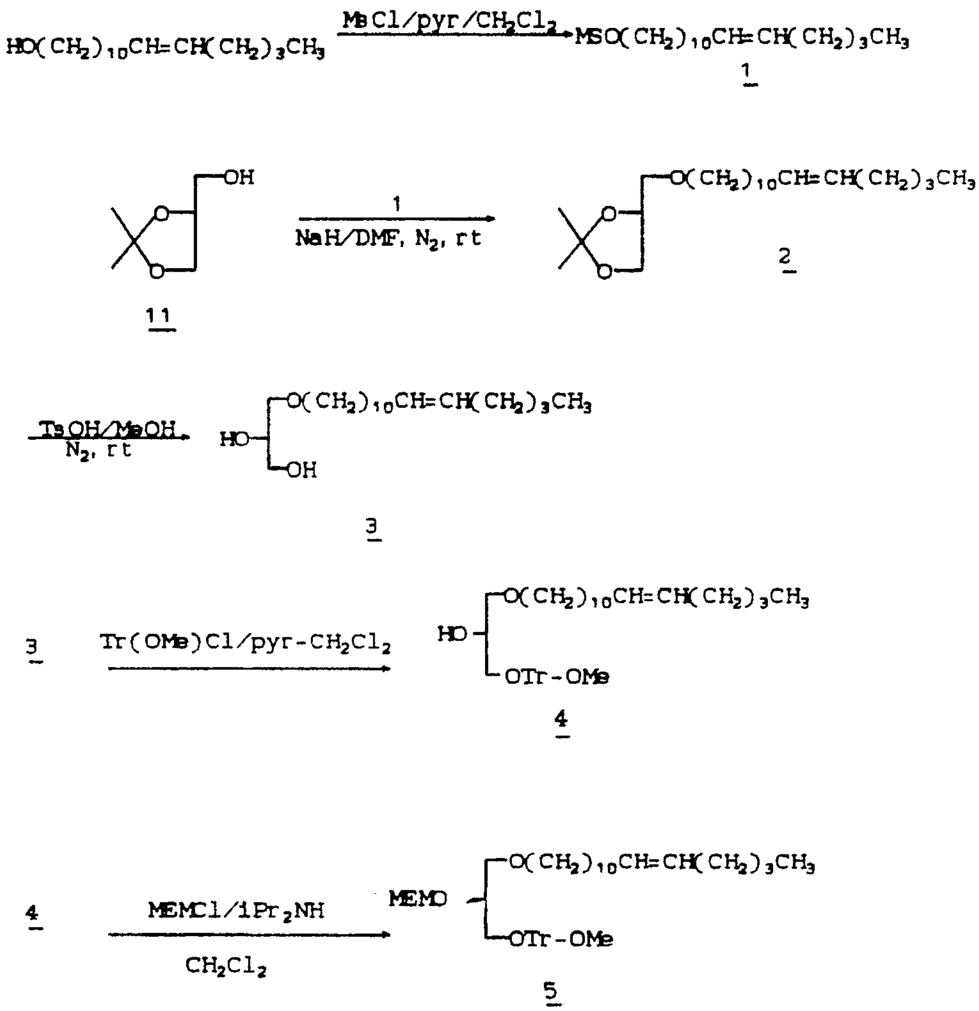
MsCl/pyr/$CH_2Cl_2$
1
11
1
NaH/DMF, $N_2$, rt
2
TsOH/MeOH
$N_2$, rt
3
3
Tr(OMe)Cl/pyr-$CH_2Cl_2$
4
4
MEMCl/iPr$_2$NH
$CH_2Cl_2$
5

SCHEME 1 (Cont'd)

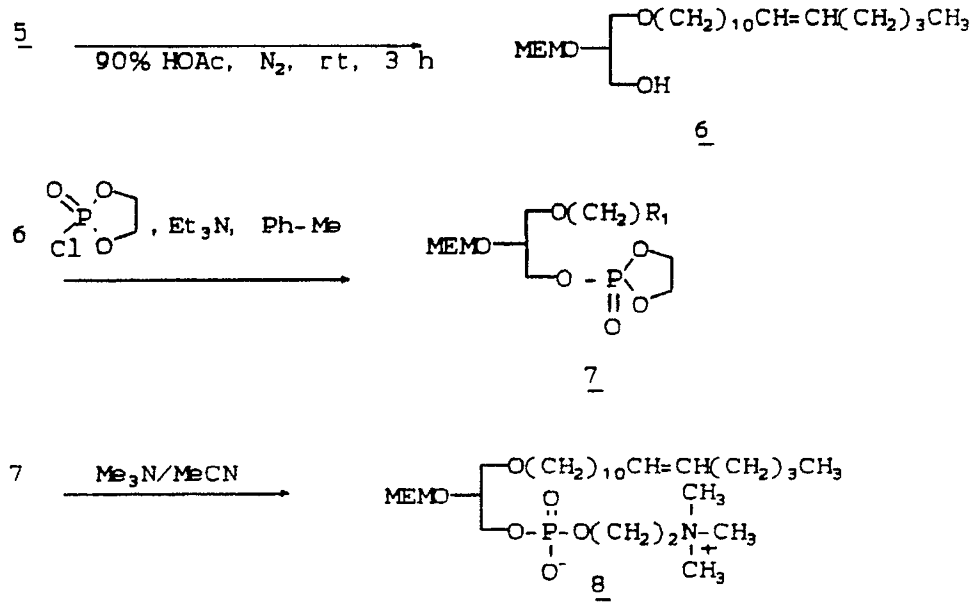


5 → 6

6 → 7

7 → 8

SCHEME 1 (Cont'd)

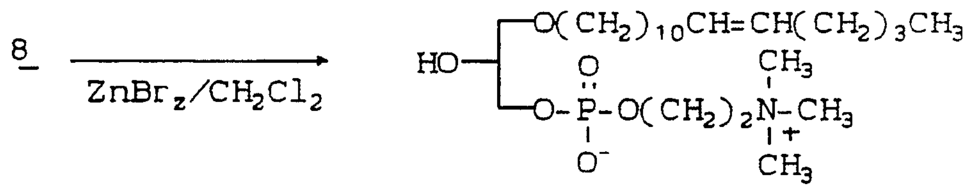


9

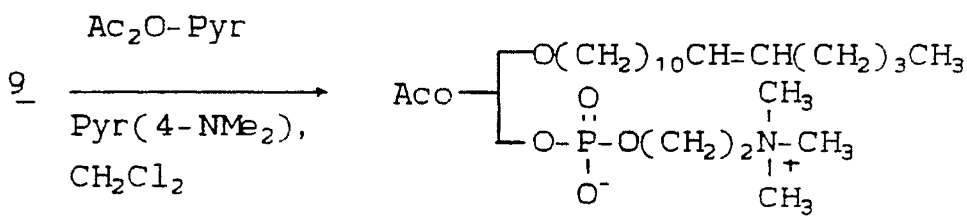


10

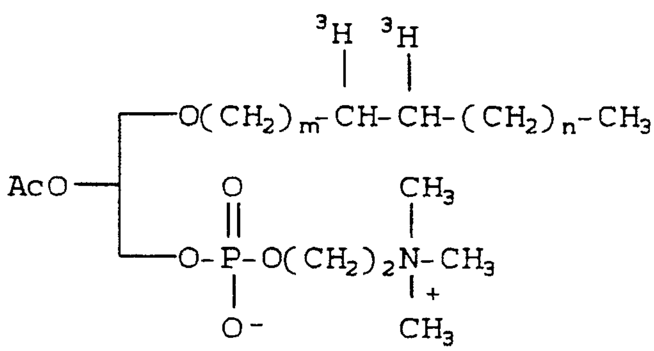

I

In the first step, cis-11-hexadecen-1-ol is converted into Cis-11-hexadecen-1-0-mesylate or 1-0-tosylate by reaction with such reagents as methanesulfonyl chloride or P-toluenesulfonyl chloride. Thereafter, compound J may be reacted with 2,3-O-isopropylidene-L-glycerol or other suitably protected glycerol to form compound A which after deprotection results in compound B. Reaction of compound B with 4-methoxytrityl chloride or trityl chloride results in compound C. Reaction of compound C with methoxyethoxymethyl chloride or methoxymethyl chloride results in compound D, which may be subsequently deprotected by addition of glacial acetic acid or other suitable mild acids. The alcohol E is then reacted with 2-chloro-2-oxo-1,3-dioxa-2-phospholane followed by trimethylamine or other conventional methods such as:

A. reaction with (2-bromoethoxy)phosphonic dichloride followed by displacement of the bromide with trimethylamine, or

B. reaction with phosphorous oxychloride followed by choline tosylate and then water,

as to result in the phosphocholine F. After deprotection of the 2-protecting group, reaction of the unsaturated lyso-PAF with acetic anhydride results in the compound H. Tritiation of the unsaturated alkenyl group results in compound I. See C.A. Demopoulos et al, J. Biol. Chem., 254, 9355-9358 (1979).

The following examples illustrate the preparaton of $[^3H]C_{16}$-PAF and intermediates thereof and as such are not to be considered as limiting the invention as set forth in the claims appended hereto:

As will be appreciated by those of skill in the art, the starting material $X(CH_2)R_1$ is readily made from the corresponding alcohol $(CH_2OH)R_1$.

Many of these alcohols or their corresponding acids are available from commercial sources. As is well appreciated the acids can be converted to the alcohol via standard reduction as with Lithium Alumimun Hydride in THF.

Additional alcohols are available via reaction of the available penta through octa - decynoic acid with Lindlars catalyst. See Dictionary of Organic Compounds, 5th edition (1982) and 4th Supplement (1986) which is replete with such alcohols and acids.

Finally as will be appreciated by those of skill in the art, any of the required alcohols can be made by a simple though tedious procedure, from a corresponding halide having one carbon less than desired via Grignard addition to formaldehyde.

EXAMPLE 1

1-O-Methylsulfonyl-cis-11-hexadecene (1)

Methanesulfonyl chloride (2.42 mL, 31.2 mmol) was added dropwise under nitrogen to a solution of cis-11-hexadecen-1-ol (5 g, 20.8 mmol) in dry dichloromethane (10 mL) and pyridine (10 mL) at 0-5 °C. The mixture was stirred under nitrogen at room temperature for 5 hours, and partitioned between dichloromethane and water. The organic layer was separated and washed successively with 2N HCl, aq. $NaHCO_3$ and aq. NaCl, dried, and evaporated to yield Compound 1 as a syrup. This material was used directly in the next experiment without further purification.

EXAMPLE 2

1-O-(cis-11-Hexadecenyl)-2,3-O-isopropylidene-L-glycerol (2)

A solution of 1-O-methylsulfonyl-cis-11-hexadecene (about 20.8 mmol, from previous experiment) in DMF (5 mL) was added under nitrogen to a suspension of 2,3-O-isopropylidene-L-glycerol (3.0 g, 22.7 mmol; $[\alpha]_D$-13.7°) and NaH (1.08 g, 27 mmol; 60% dispersion in mineral oil) in DMF (20 mL) at 0-5 °C. The reaction mixture was stirred under nitrogen at room temperature overnight, and methanol (2 mL) was added to quench excess NaH. Hexane and cold water were added and the aqueous layer was re-extracted three times with hexane. The hexane extracts were combined and washed with water, dried, and evaporated to yield Compound 2 as a brown syrup. The crude product was purified by flash-column chromatography with hexane-ethyl acetate (95:5, v/v) as the eluant. Fractions containing the desired product were combined and evaporated to an oil: $[\alpha]_D$-7.7$^0$ (c 2.23, $CHCl_3$); MS, m/z 509 [$M.^+$ + 1 + matrix (154)].

EXAMPLE 3

1-O-(cis-11-Hexadecenyl)L-glycerol (3)

p-Toluenesulfonic acid monohydrate (0.45 g, 2.37 mmol) was added to a solution of 1-O-cis-11-hexadecenyl-2,3-O-isopropylidene-L-glycerol(4.5 g, 12.7 mmol) in methanol (50 mL). The mixture was swirled and kept under nitrogen at room temperature for 30 hours. The solution was evaporated in vacuo to an oil, which was partitioned between dichloromethane and water. The organic layer was separated and washed with aq. $NaHCO_3$ and water, dried, and evaporated to dryness. The crude product was purified by flash-column chromatography with hexane ethyl acetate (3:1 followed by 2:1, v/v) as the eluant. Compound 3 was isolated as an oil (3.68 g, 92%):$[\alpha]_D$ +2.1° (c 1.89, $CHCl_3$); MS, m/z 469 [$M.^+$ + 1 + matrix (154)].

EXAMPLE 4

1-O-(cis-11-Hexadecenyl)-3-O-(4-methoxytrityl)-L-glycerol (4)

4-Methoxytrityl chloride (4.28 g, 13.86 mmol) was added in portions to a solution of 1-O-cis-11-hexadecenyl-L-glycerol (3.63 g, 11.54 mmol) in dicloromethane (15 mL) and pyridine (15 mL). The mixture was stirred under nitrogen at room temperature for 3 hours and diluted with dichloromethane. The solution was washed with 2N HCl, aq. $NaHCO_3$ and water, dried, and evaporated to dryness. TLC indicated the formation of a minor amount of di-(4-methoxytrityl) derivative as well as the desired mono-(4-methoxytrityl)

compound. Compound 4 was isolated by flash-column chromatography with hexane ethyl acetate-$Et_3N$ (95:5:0.1%, v/v) as the eluant. This material was used directly in the next experiment without further purification.

EXAMPLE 5

1-O-(cis-11-Hexadecenyl)-2-O-[(methoxyethoxy)methyl]-3-O-(4-methoxytrityl)-L-glycerol (5)

Methoxyethoxymethyl chloride (2.8 mL, 24.5 mmol) was added dropwise to a solution of 1-O-(cis-11-hexadecenyl)-3-O-(4-methoxytrityl)-L-glycerol (7.19 g, from previous experiment) in dichloromethane (25 mL) containing diisopropylethylamine (4.30 mL, 24.5 mmol). The solution was stirred under nitrogen at room temperature overnight. It was diluted with dichloromethane and washed with water, dried, and evaporated to dryness to yield Compound 5. The crude product was put on a flash-column of silica gel and eluted with hexane-ethyl acetate (90:10, v/v). Fractions containing the title compound were combined and evaporated to dryness: MS, m/z 273 (4-methoxytrityl+).

EXAMPLE 6

1-O-(cis-11-Hexadecenyl)-2-O-[(methoxyethoxy)methyl]-L-glycerol (6)

A solution of 1-O-(cis-11-hexadecenyl)-2-O-[(methoxyethoxy)methyl]-3-O-(4-methoxytrityl)-L-glycerol (5.0 g, 7.41 mmol) in 90% glacial acetic acid (50 mL) was kept under nitrogen at room temperature for 3 hours and evaporated to dryness. The resulting oil was taken up in dichloromethane and the solution was washed with aq. $NaHCO_3$ and water, dried, and concentrated to dryness. The residue was put on a flash-column of silica gel and eluted with hexane-ethyl acetate (85:15 followed by 80:20, v/v). The desired fractions were combined and evaporated to yield Compound 6 (2.47 g, 83%): $[\alpha]_D$-28.5° (c 1.79, $CHCl_3$); MS, m/z 557 [$M^+$ + 1 + matrix (154)], 481 ($M^+$-$CH_3OCH_2CH_2\bar{O}$).

EXAMPLES 7 and 8

1-O-(cis-11-Hexadecenyl)-2-O-[methoxylthoxylmethyl]-sn-glycero-3 phosphocholorine (8)

2-Chloro-2-oxo-1,3-dioxa-2-phospholane (0.72 mL, 5.2 mmol) was added under nitrogen to a solution of 1-O-(cis-11-hexadecenyl)-2-O-[(methoxyethoxy)methyl]-L-glycerol (2.1 g, 5.2 mmol) and triethylamine (0.75 mL, 5.2 mmol) in dry toluene (8 mL) at 0-5° C. The reaction mixture was warmed to room temperature and stirred under nitrogen for an additional 2 hours. Triethylammonium chloride was filtered off and washed with toluene. The combined filtrates were evaporated give the cyclic phosphate triester 7.

Compound 7 was dissolved in dry acetonitrile (15 mL) and trimethylamine (1.63 mL) was added. The mixture was heated in a pressurized bottle at 65° C overnight. The solution was evaporated to dryness and the residue was put on a flash-column of silica gel and eluted with $CHCl_3$-MeOH-30% $NH_4OH$ (1:9:1, v/v). The more mobile fractions (negative to molybdenum blue) were combined, evaporated, and re-purified by flash-column chromatography with hexane-ethyl acetate (4:1, v/v) as the eluant. This gave back the starting material, 1-O-(cis-11-hexadecenyl)-2-O-[(methoxyethoxy)methyl]-L-glycerol(312 mg). The less mobile fractions from the first column, which contained the desired product (positive to molybdenum blue) were combined and evaporated to dryness to yield Compound 8 $[\alpha]_D$ +9.2° (c 1.64, $CHCl_3$); MS, m/z 722 [$M^+$ + 1 + matrix (154)], m/z 184 [$HOPO(OH)OCH_2CH_2N+NMe_3$].

EXAMPLE 9

1-O-(cis-11-Hexadecenyl)-sn-glycero-3-phosphocholine (9)

Anhydrous zinc bromide (1.43 g, 6.36 mmol) was added to a solution of 1-O-(cis-11-hexadecenyl)-2-O-[-(methoxyethoxy)methyl]sn-glycero-3-phosphocholine (0.72 g, 1.27 mmol) in dichloromethane (10 mL) and the mixture was stirred under nitrogen at room temperature overnight. Chloroform-methanol-water (1:0.9:1, v/v; 30 mL) was added and the organic layer was separated. The aqueous layer was re-extracted with chloroform-methanol (1:0.9, v/v; 20 mL). The organic extracts were combined, dried, and evaporated to dryness. The crude product (0.61 g) was put on a flash-column of a silica gel and eluted with $CHCl_3$-MeOH-30% $NH_4OH$ (1:9:1, v/v). This gave the starting material (86 mg) and the Compound 9: (0.35g, 65% based

on the used starting material) $[\alpha]_D$-6.9° (c 1.61, $CHCl_3$); MS, m/z 634 [$M^+$ + 1 + matrix (154)], m/z 184 [$HOPO(OH)OCH_2CH_2N+NMe_3$].

EXAMPLE 10

1-O-(cis-11-Hexadecenyl)-2-O-acetyl-sn-glycero-3-phosphocholine (10)

Acetic anhydride (9 mL) was added to a suspension of 1-O-(cis-11-hexadecenyl)-sn-glycero-3-phosphocholine (370 mg, 0.77 mmol), 4-(N,N-dimethylamino)pyridine (94 mg, 0.77 mmol), and pyridine (0.9 mL) in dichloromethane (5 mL) at 0-5°C. The mixture was stirred under nitrogen at room temperature overnight and the solution was evaporated in vacuo. Excess acetic anhydride was co-distilled several times with methanol. The residue was put on a flash-column of silica gel and eluted with $CHCl_3$-MeOH-$H_2O$ (65:35:6, v/v). The title compound was isolated as a foam: $[\alpha]_D$. 1.5° (c 1.37, $CHCl_3$); MS, m/z 676 [$M^+$ + 1 + matrix (154)], m/z 184 [$HOPO(OH)OCH_2CH_2N+NMe_3$].

## Claims

1. A process of making [$^3H$]-- labelled PAF of the formula:

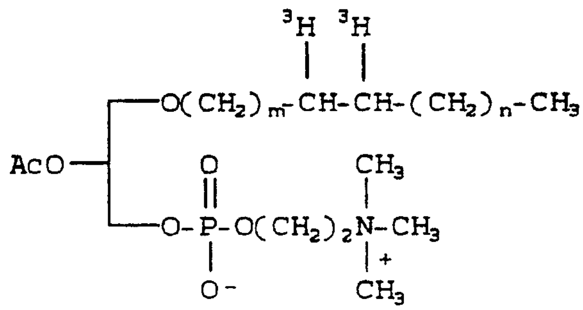

where m and n are positive integers and the sum of m + n is from 12 to 15, comprising the steps of (A) contacting a compound of formula G

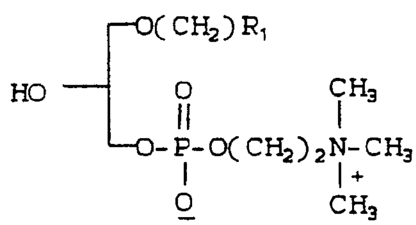

G

wherein $R_1$ is $C_{14-17}$ alkenyl, in dichloromethane organic solvent and in the presence of a base with acetic anhydride to yield a compound of formula H, and;

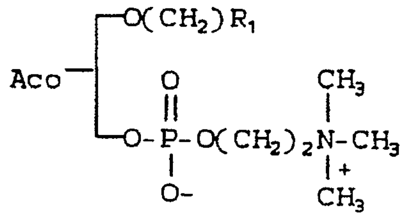

H

(B) tritiating the compound of formula H at the point of unsaturation in the alkenyl group $R_1$, to yield a compound of formula I,

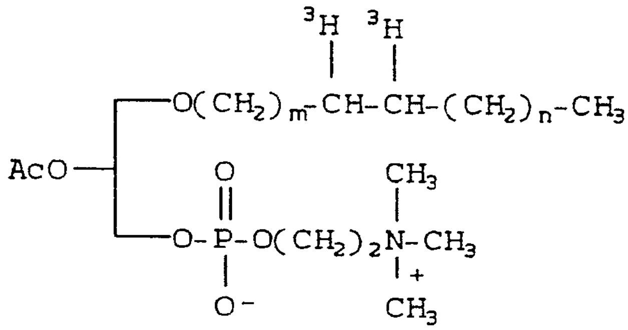

I

2. A process of making [$^3$H] - labelled PAF of the formula:

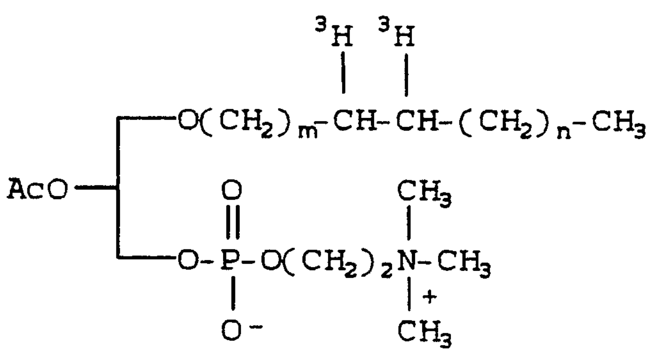

according to Claim 1
wherein m and n are positive integers and the sum of m + n is from 12 to 15,

comprising the steps of :

(A) contacting a compound of formula J

$X(CH_2)R_1$ J

wherein:

$R_1$ is $C_{14-17}$alkenyl,

X is a first leaving group,

in a first aprotic solvent, with a strong base and 2,3-O-isopropylidene-L-glycerol to yield a compound

of formula A,

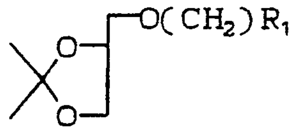

A

(B) contacting a compound of formula A, in $C_{1-8}$ alkanol, with a strong acid to yield a compound of formula B,

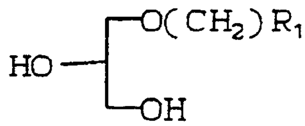

B

(C) contacting a compound of formula B, in a second aprotic solvent in the presence of a second base, with 4-methoxytrityl chloride or trityl chloride to yield a compound of formula C,

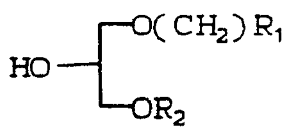

C

wherein $R_2$ is an acid labile protecting group;

(D) contacting a compound of formula C, in an organic solvent and in the presence of a third base, with methoxyethoxymethyl chloride or methoxymethyl chloride to yield a compound of formula D,

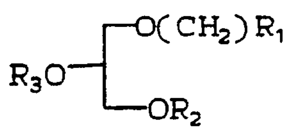

D

wherein $R_3$ is a protecting group, labile at pH 6-9.

(E) contacting a compound of formula D with a second acid to yield a compound of formula E,

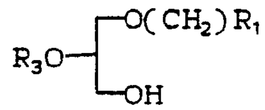

E

(F) converting a compound of formula E to yield a compound of formula F,

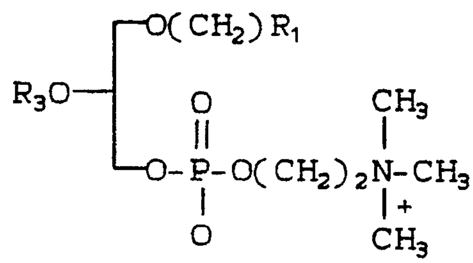

F

(G) contacting a compound of formula F, in a halogenated organic solvent, with $ZnBr_2$ or $TiCl_4$ to yield a compound of formula G,

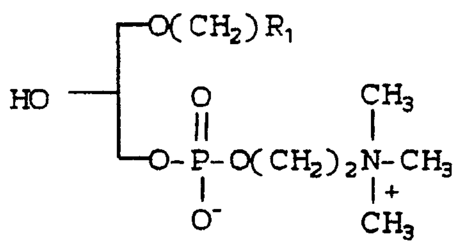

G

(H) contacting a compound of formula G, in a halogenated organic solvent and in the presence of a fourth base with acetic anhydride to yield a compound of formula H, and

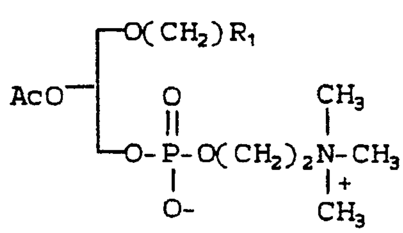

H

and;

(I) tritiating the compound of formula H at the point of unsaturation in the alkenyl group $R_1$ to yield a compound of formula I,

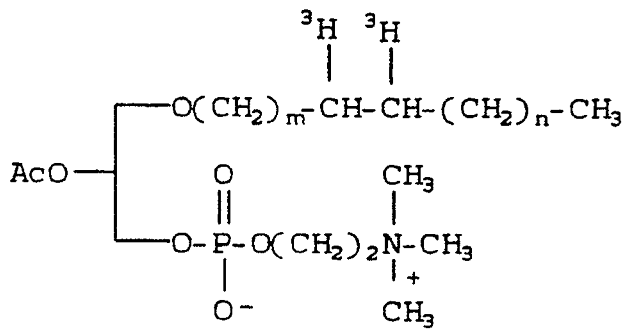

I

3. A process according to Claim 2 wherein m is 10 and n is 3.

4. The process according to Claim 2 wherein, in step (A), the leaving group is methanesulfonyloxy, or p-toluenesulfonyloxy, the first aprotic solvent is dimethylformamide and the strong base is a metallic hydride.

5. Process according to Claim 4 wherein, in step (C) of Claim 2, the acid labile protecting group is 4-methoxytrityl and, the second aprotic solvent is dichloromethane.

6. Process according to Claim 5 wherein, in step (G) of Claim 2, the halogenated organic solvent is dichloromethane, and wherein m is 10 and n is 3.

7. A process of making [$^3$H] - labelled PAF of the formula:

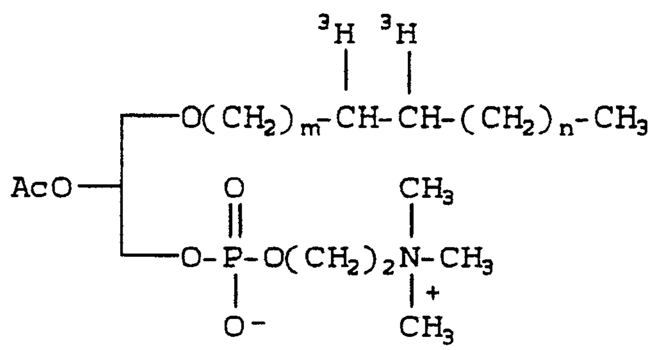

according to Claim 1
wherein m and n are positive integers and the sum of m + n is from 12 to 15,

comprising the steps of :
(A) Contacting a compound of formula J

$X(CH_2)R_1$ J

wherein:
R1 is $C_{14-17}$alkenyl,
X is a leaving group which is methanesulfonyloxy in a first aprotic solvent which is dimethyl formamide, with a strong base and 2,3-O-isopropylidene-L-glycerol to yield a compound of formula A,

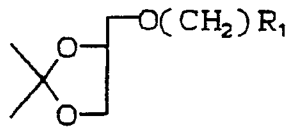

A

(B) contacting a compound of formula A, in methanol, with a strong acid to yield a compound of formula B,

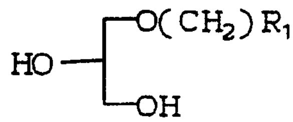

B

(C) contacting a compound of formula B, in a second aprotic solvent which is dichloromethane in the presence of a second base, with 4-methoxytrityl chloride to yield a compound of formula C,

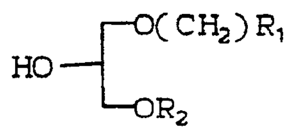

C

wherein $R_2$ is a 4-methoxytrityl group;
(D) contacting a compound of formula C, in a halogenated organic solvent and in the presence of a third base, with methoxyethoxymethyl chloride to yield a compound of formula D,

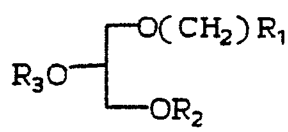

D

wherein $R_3$ is methoxyethoxymethyl;
(E) contacting a compound of formula D with acetic acid to yield a compound of formula E,

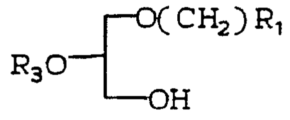

E

(F) Converting a compound of formula E to yield a compound of formula F,

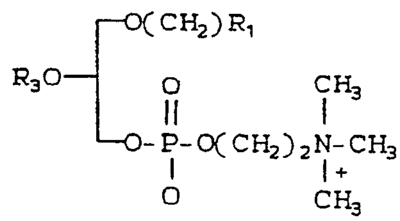

F

(G) contacting a compound of formula F, in dichloromethane solvent, with $ZnBr_2$ to yield a compound of formula G,

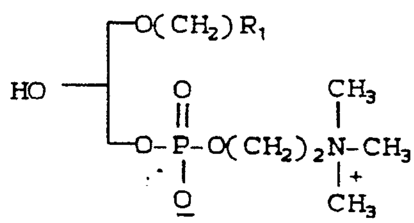

G

(H) contacting a compound of formula G, in dichloromethane organic solvent and in the presence of a fourth base with acetic anhydride to yield a compound of formula H,

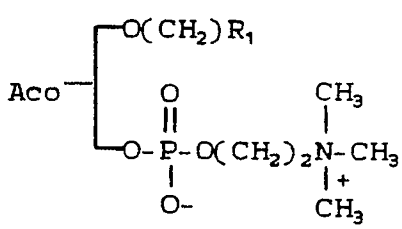

H

and;
(I) tritiating the compound of formula H at the point of unsaturation in the alkenyl group $R_1$, to yield a compound of formula I,

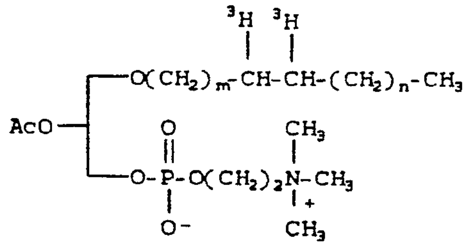

I

8. A compound selected from the group consisting of:

(a) A compound of formula A which is

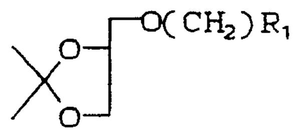

A

(b) A compound of formula B which is

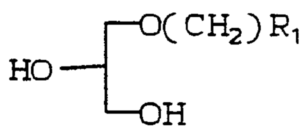

B

wherein $R_1$ is $C_{14-17}$alkenyl, or

(c) A compound of formula C which is

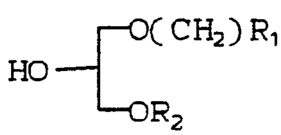

C

wherein $R_1$ is $C_{14-17}$alkenyl, and $R_2$ is 4-methoxytrityl or trityl.

9. A compound selected from the group consisting of:

(a) A compound of the formula D which is

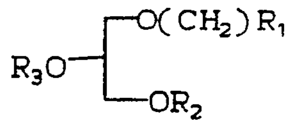

D

wherein $R_1$ is $C_{14-17}$alkenyl, $R_2$ is 4-methoxytrityl or trityl, and $R_3$ is methoxyethoxymethyl or methoxymethyl,

(b) A compound of formula E which is

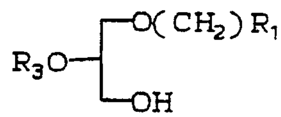

E

wherein $R_1$ is $C_{14-17}$alkenyl, and $R_3$ is methoxyethoxymethyl or methoxymethyl.

(c) A compound of formula E' which is

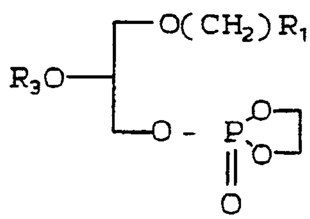

E'

wherein $R_1$ is $C_{14-17}$alkenyl and $R_3$ is methoxyethoxymethyl or methoxymethyl,

(d) A compound of formula F which is

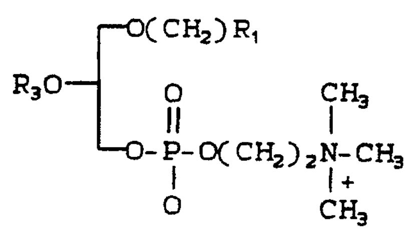

F

wherein $R_1$ is $C_{14-17}$alkenyl, and $R_3$ is methoxyethoxymethyl or methoxymethyl, or

(e) A compound of formula G which is

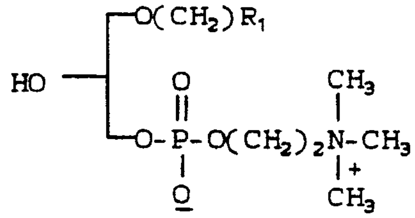

G

wherein $R_1$ is $C_{14-17}$alkenyl.

**10.** A compound of formula H which is:

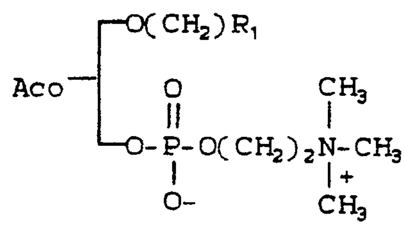

H

wherein $R_1$ is $C_{14-17}$alkenyl, with the proviso that the compound of formula H is other than $\Delta^{9,10}$-[$^3$H]$C_{16}$-PAF, $\Delta^{9,10}$-[$^3$H]$C_{18}$-PAF or $\Delta^{1,2}$-[3H]$C_{16}$-PAF.

**11.** A compound of formula I which is:

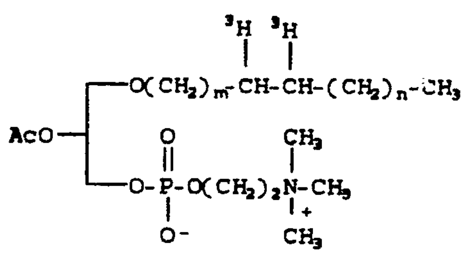

I

wherein m and n are positive integers and the sum of n + m is from 12 to 15 with the proviso that the compound of formula I is other than $\Delta^{9,10}$ -[$^3$H]$C_{16}$-PAF, $\Delta^{9,10}$-[$^3$H]$C_{18}$-PAF or $\Delta^{1,2}$-[$^3$H]$C_{16}$-PAF.